Europäisches Patentamt

European Patent Office

Office européen des brevets

(11) Publication number: **0 445 659 A2**

# EUROPEAN PATENT APPLICATION

(21) Application number: 91103039.3

(51) Int. Cl.5: **A61K 7/11, A61K 7/06**

(22) Date of filing: 28.02.91

(30) Priority: 09.03.90 JP 59488/90

(43) Date of publication of application:
**11.09.91 Bulletin 91/37**

(84) Designated Contracting States:
**AT CH DE LI NL**

(71) Applicant: KAO CORPORATION
**1-14-10, Nihonbashikayaba-cho**
**Chuo-ku Tokyo(JP)**

(72) Inventor: **Suzuki, Noboru**
**3-19-8, Kitakoiwa**
**Edogawa-ku, Tokyo(JP)**
Inventor: **Andoh, Yohji**
**1-13-11, Takanodai**
**Funabashi-shi, Chiba-ken(JP)**

(74) Representative: **Wächtershäuser, Günter, Dr.**
**Tal 29**
**W-8000 München 2(DE)**

(54) **Hair-set composition.**

(57) A hair-set composition is disclosed. The composition comprises (a) 0.05-10.0% by weight of carageenan and (b) 0.01-30.0% by weight of an ethylene oxide-addition type nonionic surface active agent which is a transparent liquid or a transparent paste at 50°C and solid at 25°C. It is useful for hair-setting or hair-styling and can be prepared into a foaming type, a jelly type, a pump spray type, an aerosol mist type, or a lotion type preparation. The hair-set composition imparts no sticky feeling throughout a hair-set operation, allows the hair to be easily combed without hardness, and exhibits excellent hair-set retentivity.

## BACKGROUND OF THE INVENTION

Field of the Invention:

This invention relate to a hair-set composition, and, more particularly, to a hair set composition providing superior hair-set retentivity and excellent feeling upon use. The composition is useful for hair-setting, hair-styling, and like.

Description of the Background Art:

Most conventional hair-set compositions widely used are solutions of film-forming polymeric compounds in solvents such as water, lower alcohols, or their mixtures. The polymeric compounds used in such compositions have a function of fixing hairs and retaining the set hair. The polymeric compounds, however, impart a sticky feeling while the hair is being set and are not necessarily satisfactory in styling the hair. In addition, they make the hair hard after setting, providing unfavorable feeling to the touch.

In order to eliminate these drawbacks, attempts have been undertaken to prepared hair-set compositions in which a certain cosmetic oil or a surfactant is used together with a polymeric compound (Japanese Patent Laid-open Nos. 153609/89, 132680/89, etc.). Such attempts, however, have not been successful in providing a hair-set composition which satisfies the demanded hair-set retentivity and a good feeling to the touch.

There has existed a strong desire for the development of hair-set compositions which can provide an adequate adhesion force between the hairs, produce an elastic and soft film, and provide no sticky feeling throughout hair-set operation from the application of the composition to drying and finishing, and yet properly retain the style of the set hair.

## SUMMARY OF THE INVENTION

In view of this situation, the present inventors have undertaken extensive studies and have found that a hair set composition providing superior hair-set retentivity and an excellent feeling during the hair-set could be produced by incorporating a specific ethylene oxide-addition type nonionic surface active agent to carageenan. This finding has led to the completion of the present invention.

Accordingly, an object of the present invention is to provide a hair-set composition comprising (a) 0.05-10.0% by weight of carageenan and (b) 0.01-30.0% by weight of an ethylene oxide-addition type nonionic surface active agent which is a transparent liquid or a transparent paste at $50°$ C and solid at $25°$ C.

In a preferred embodiment of the present invention, said ethylene oxide-addition type nonionic surface active agent is a member selected from the group consisting of polyoxyethylene alkyl ethers, polyoxyethylene hydrogenated castor oils, polyethyleneglycol mono-fatty acid esters, and polyoxyethylene glyceryl mono-fatty acid esters.

Other and further objects, features and advantages of this invention will appear more fully from the following description.

## DETAILED DESCRIPTION OF THE INVENTION AND PREFERRED EMBODIMENTS

Any one of *Kappa, Iota, and Lambda* carageenans can be used in the present invention. Especially preferable are *Iota, and Lambda* carageenans. They can be used either independently or in combination. The amount to be incorporated into the composition is usually 0.05-10.0% by weight, and preferably 0.2-5.0% by weight. If the amount is smaller than 0.05% by weight, no hair-set effect can be obtained. The amount greater than 10% by weight of carageenan in the composition is too much to be applied to the hair, leading to problems in washing the hair.

Ethylene oxide-addition type nonionic surface active agent which is component (b) in the composition of the present invention is that which is a transparent liquid or a transparent paste at $50°$ C and solid at $25°$ C. That is to say, component (b) has a melting point higher than $25°$ C but lower than $50°$ C and a viscosity smaller than 100,000 poise at $50°$ C. Those which are solid at $50°$ C provide only an insufficient adhesion force between hairs, resulting in an inadequate hair-set effect. Those which are a transparent liquid or a transparent paste at $25°$ C imparts a sticky feeling during hair drying and makes it difficult to properly arrange the hair.

Given as preferable examples of component (b) are polyoxyethylene (EO = 10-25) lauryl ether, polyoxyethylene (EO = 5-15) cetyl ether, polyoxyethylene (EO = 5-10) stearyl ether, polyoxyethylene (EO = 5-25) hexyldecyl ether, polyoxyethylene (EO = 8-25) octyldodecyl ether, polyoxyethylene (EO = 10-30) isostearyl ether, polyoxyethylene (EO = 10-25) decyltetradecyl ether, polyoxyethylene (EO = 10-20) decylpentadecyl ether, polyoxyethylene (EO = 25-100) hydrogenated castor oil, polyethyleneglycol (EO = 5-25) monostearate, polyoxyethylene (EO = 5-40) glyceryl monostearate, and the like. Of these, polyoxyethylene hexyldecyl ether, polyoxyethylene octyldodecyl ether, polyoxyethylene isostearyl ether, and hydrogenated castor oil are particularly preferable. These nonionic surface active agents are incorporated in the composition of the present invention in an amount of 0.01-30.0% by weight, and preferably 0.1-10.0% by weight.

A preferable ratio by weight of carageenan and nonionic surface active agent in the composition is in the range of 1:30-5:1.

Besides these essential components, various optional components can be added to the composition of the present invention to the extent that the effect of the invention is not adversely affected. Such optional components include substances for adjusting the viscosity and gel strength such as inorganic salts (e.g. sodium chloride, potassium chloride), natural or synthetic polymers (e.g. low cast bean gum, xanthane gum, cross-linked polyacrylic acid), glycerides such as avocado oil, jojoba oil, macademian nut oil, olive oil), waxes (e.g. bees wax, lanoline), hydrocarbons (e.g. liquid paraffin, isoparaffin, squalan), linear or branched higher alcohols (e.g. cetyl alcohol, stearyl alcohol, 2-octyldodecanol), polyhydric alcohols (e.g. propylene glycol, glycerol, 1,3-butylene glycol, polyglycerine, sorbitol), esters (e.g. isopropyl myristate, octyldodecyl myristate), amides (e.g. diethanolamide oleate, diethanolamide laurate), silicone derivatives (e.g. dimethylpolysiloxane, methylphenylpolysiloxane, polyether-modified silicone, amino-modified silicone), cationic surface active agents (e.g. stearyltrimethylammonium chloride, distearyldimethylammonium), anionic surface active agents (e.g. polyoxyethylenelauryl ether sulfate, polyoxyethylenelauryl sulfosuccinate), amphoteric surface active agents (e.g. laurylhydroxy sulfobetain, lauryldimethyl carbobetain), nonionic polymers (e.g. polyvinyl pyrrolidone, copolymer of vinyl pyrrolidone and vinyl acetate), anionic polymers (e.g. copolymer of acrylic or methacrylic acid and alkyl (meth)acrylate), amphoteric polymer (e.g. copolymer of N-methacryloylethyl-N,N-dimethylammonium-$\alpha$-N-methylcarboxy betain and butyl methacrylate), protein derivatives or amino acids (e.g. hydrolyzates of collagen or keratine), plant extracts, Chinese medicines, vitamins, UV absorbers (e.g. oxybenzon), preservatives (e.g. paraben), chelating agents (e.g. EDTA-Na), dyes, pigments, perfumes, and the like.

The hair-set composition of the present invention can be prepared by a conventional method by using water and/or lower alcohols depending on the types of the preparations. The composition may be of an aerosol type in which a propellant such as chlorofluoro alkanes, liquefied petroleum gases, dimethyl ether, carbon dioxide gas, or nitrogen gas is used together. Specific types of the preparations of the hair-set composition of the present invention include a foaming type, a jelly type, a pump spray type, an aerosol mist type, a lotion type, and the like.

Other features of the invention will become apparent in the course of the following description of the exemplary embodiments which are given for illustration of the invention and are not intended to be limiting thereof.

EXAMPLES

Example 1

Hair-set compositions shown in Table 1 were prepared and their hair-set retentivity and feeling and hair-style performance were evaluated.

<Method of preparation>

Components (5) and (6) were added to a solution of components (1)-(4) in which component (7) is dissolved, and the whole was blended to complete dissolution.

<Method of evaluation>

(1) Hair-set retentivity

The hair-set composition (0.3 gm) was applied to a tress of hair (length: 18 cm, weight: 1.5 gm) which had been wetted with water. The hair was wound around a rod with a 15 mm diameter and allowed to dry. After drying, the rod was released from the curled hair and the hair tress was suspended in a thermostat (25 °C, 98% RH) for 30 minutes, following which the length of the curl was measured. The hair-set

3

retentivity (%) was determined between the length of the curl immediately after the rod was removed, as 100%, and the length of the hair which had not been curled (18 cm), as 0%. The results of the evaluation were graded as follows:

```
Hair-set retentivity (%)

        0-33                    CCC

        34-66                   BBB

        67-100                  AAA
```

(2) Feeling and hair-style performance

A human hair wig of which the hairs are arranged in the same direction from the root to the end was wetted with water. Ten (10) expert hairdressers set the hair using 2 gm of the hair-set composition for each hair wig to organoleptically evaluate the easiness of hair-set.
The following standard was applied to the evaluation.

```
        Very good                +3

        Good                     +2

        Better than normal       +1


        Normal                    0

        Worse than normal        -1

        Bad                      -2

        Very bad                 -3
```

The average of the scores was graded as follows to express the results of the evaluation.

```
    -3 to -2 (-2 is excluded)       FFF

    -2 to -1 (-1 is excluded)       EEE


    -1 to 0 (0 is excluded)         DDD

     0 to +1 (+1 is excluded)       CCC

    +1 to +2 (+2 is excluded)       BBB

    +2 to +3                        AAA
```

TABLE 1

| Component (%) | Comparative Composition | | | | | Invention Composition |
|---|---|---|---|---|---|---|
| | 1 | 2 | 3 | 4 | 5 | 1 |
| (1) Copolymer of acrylic acid/methacrylic acid ester *1 | 6.0 | - | 6.0 | - | - | - |
| (2) Polyvinyl alcohol | - | - | - | - | 1.8 | - |
| (3) Lambda carageenan | - | 1.8 | - | 1.8 | - | 1.8 |
| (4) Polyoxyethylene (EO=10) stearyl ether | - | - | 0.8 | - | 0.8 | 0.8 |
| (5) Ethanol | 15.8 | 20.0 | 15.8 | 20.0 | 20.0 | 20.0 |
| (6) Propylene glycol | - | - | - | 5.0 | - | - |
| (7) Water | | | Balance | | | |
| Evaluation | | | | | | |
| Hair-set retentivity | BBB | BBB | BBB | BBB | CCC | AAA |
| Stickilessness during hair-set and drying | FFF | DDD | DDD | CCC | EEE | AAA |
| Easiness of hair-set | DDD | EEE | DDD | EEE | DDD | AAA |
| Non-hardness after hair-set | FFF | EEE | DDD | DDD | DDD | AAA |

*1 30% ethanol solution of Plas-size L53P (Trademark, manufacture by Goou Chemical)

As clear from the results shown in Table 1, the hair-set composition of the present invention exhibited excellent hair-set retentivity and gave a good feeling upon use.

Example 2

Hair-set compositions shown in Table 2 were prepared and their hair-set retentivity and feeling upon use were evaluated according to the same methods and evaluation standard as in Example 1. The results are shown in Table 2.

<Method of preparation>

Components (1) and (2)-(11) were added to water (13). After heating with stirring, and the solution was cooled and component (12) was added.

TABLE 2

| Component (%) | Invention Composition | | | | Comparative Composition | | | | | |
|---|---|---|---|---|---|---|---|---|---|---|
| | 2 | 3 | 4 | 5 | 6 | 7 | 8 | 9 | 10 | 11 |
| (1) Lambda carageenan | 0.8 | 0.8 | 0.8 | 0.8 | 0.8 | 0.8 | 0.8 | 0.8 | 0.8 | 0.8 |
| (2) Polyethyleneglycol (EO=10) monostearate | 0.8 | - | - | - | - | - | - | - | - | - |
| (3) Polyoxyethylene (EO=15) lauryl ether | - | 0.8 | - | - | - | - | - | - | - | - |
| (4) Polyoxyethylene (EO=20) glyceryl monostearate | - | - | 0.8 | - | - | - | - | - | - | - |
| (5) Polyoxyethylene (EO=15) decyltetradecyl ether | - | - | - | 0.8 | - | - | - | - | - | - |
| (6) Polyethyleneglycol (EO=40) monostearate *1 | - | - | - | - | 0.8 | - | - | - | - | - |
| (7) Polyoxyethylene (EO=4) lauryl ether *2 | - | - | - | - | - | 0.8 | - | - | - | - |
| (8) Polyoxyethylene (EO=40) lauryl ether *1 | - | - | - | - | - | - | 0.8 | - | - | - |

TABLE 2 (Continued)

| Component (%) | Invention Composition | | | | Comparative Composition | | | | | |
|---|---|---|---|---|---|---|---|---|---|---|
| | 2 | 3 | 4 | 5 | 6 | 7 | 8 | 9 | 10 | 11 |
| (9) Polyoxyethylene (EO=40) stearyl ether *1 | - | - | - | - | - | - | - | 0.8 | - | - |
| (10) Polyethyleneglycol (EO=250) distearate *1 | - | - | - | - | - | - | - | - | 0.8 | - |
| (11) Polyethyleneglycol (EO=5) . decyltetradecyl ether *2 | - | - | - | - | - | - | - | - | - | 0.8 |
| (12) Ethanol | 15.0 | 15.0 | 15.0 | 15.0 | 15.0 | 15.0 | 15.0 | 15.0 | 15.0 | 15.0 |
| (13) Water | Balance | | | | | | | | | |
| Evaluation | | | | | | | | | | |
| Hair-set retentivity | AAA | AAA | AAA | AAA | CCC | BBB | CCC | CCC | CCC | CCC |
| Stickilessness during hair-set and drying | AAA | AAA | AAA | AAA | DDD | FFF | CCC | DDD | DDD | EEE |
| Easiness of hair-set | AAA | AAA | AAA | AAA | CCC | EEE | DDD | DDD | DDD | DDD |
| Non-hardness after hair-set | AAA | AAA | AAA | AAA | DDD | DDD | CCC | CCC | CCC | DDD |

*1 : Solid at 50°C
*2 : Transparent liquid at 25°C

As can bee seen from Table 2, ethylene oxide-addition type nonionic surface active agents which are solid at 50° C or a transparent liquid at 25° C do not provide good results in hair-set retentivity nor do they favorably evaluated in the organoleptic evaluation. On the other hand, the hair-set compositions of the present invention exhibited superior hair-set retentivity and an excellent feeling in terms of both stickilessness and softness.

Example 3 Styling foam

| Component | (%) |
|---|---|
| (1) *Lambda* carageenan | 2.0 |
| (2) Polyoxyethylene (EO=20) hexyldecyl ether | 0.6 |
| (3) Amino-modified silicone (SM8702C, a product of Toray Silicone Co.) | 0.2 |
| (4) Disodium Polyoxyethylenelauryl sulfosuccinate (EO=3) | 0.4 |
| (5) Plas-size L53P | 4.0 |
| (6) Propylene glycol | 0.5 |
| (7) Ethanol | 5.0 |
| (8) Perfume | 0.2 |
| (9) Water | Balance |
| (10) LPG ($3.5 \text{ kg/cm}^2\text{G}$, 20°C) | 8.0 |
| Total | 100.0 |

<Method of preparation>

Component (1) was added to component (9) and heated to dissolution. To this solution components (2)-(8) and (10) were added. The product thus prepared was filled in aerosol containers to obtain the styling foam. The product imparted no sticky feeling throughout hair-setting operation, allowed the hair to be easily combed without hardness, and exhibited excellent hair-set retentivity.

Example 4 Styling jelly

| Component | (%) |
|---|---|
| (1) *Iota* carageenan | 1.0 |
| (2) Polyoxyethylene (EO=15) lauryl ether | 0.5 |
| (3) Carbopole 940 (polymerized acrylic acid, a product of B.F. Goodrich Chemical Co.) | 0.6 |
| (4) Polyvinyl pyrrolidone (Luviskol K-90, BASF) | 0.5 |
| (5) Triethanolamine | q.s. (pH 7.5) |
| (6) Glycerol | 2.0 |
| (7) Keratine hydrolyzate | 0.5 |
| (8) Perfume | 0.1 |
| (9) Oxybenzone | 0.1 |
| (10) Pigment | Small amount |
| (11) Ethanol | 25.0 |
| (12) Water | Balance |
| Total | 100.0 |

<Method of preparation>

Components (1), (3) and (4) were added to water and heated to dissolution. To this solution components (2) and (5)-(11) were added to produce a styling jelly. The product imparted no sticky feeling throughout hair-set operation, allowed the hair to be easily combed with no hardness, and exhibited excellent hair-set retentivity.

Example 5 Styling jelly (wet type)

| Component | (%) |
|---|---|
| (1) *Kappa* carageenan | 0.3 |
| (2) *Iota* carageenan | 0.3 |
| (3) Polyoxyethylene (EO=80) hydrogenated castor oil | 10.0 |
| (4) Glycerol | 30.0 |
| (5) Carbopole 940 (product of B.F. Goodrich Chemical Co.) | 0.4 |
| (6) Triethanolamine | q.s. (pH 7.5) |
| (7) Dimethylpolysiloxane (5,000 cs) | 0.5 |
| (8) Polyether-modified silicone (L-720, Nippon Uniker Co.) | 0.4 |
| (9) Birch Extract (product of Novarom GmbH) | 0.3 |
| (10) Perfume | 0.1 |
| (11) Ethanol | 10.0 |
| (12) Silk protein hydrolyzate | 0.3 |
| (13) Water | Balance |
| Total | 100.0 |

<Method of preparation>

Components (1), (2) and (5) were added to water and heated to dissolution. To this solution components (3), (4) and (6)-(12) were added to produce a wet type styling jelly. The product imparted no sticky feeling throughout a hair-set operation, allowed the hair to be easily combed without hardness, and exhibited excellent hair-set retentivity.

Obviously, numerous modifications and variations of the present invention are possible in light of the above teachings. It is therefore to be understood that within the scope of the appended claims, the invention may be practiced otherwise than as specifically described herein.

**Claims**

1. A hair-set composition characterized by comprising (a) 0.05-10.0% by weight of carageenan and (b) 0.01-30.0% by weight of an ethylene oxide-addition type nonionic surface active agent which is a transparent liquid or a transparent paste at 50°C and solid at 25°C.

2. The hair-set composition according to Claim 1, wherein said carageenan is a member selected from the group consisting of *Kappa, Iota, and Lambda* carageenans.

3. The hair-set composition according to Claim 1, wherein said carageenan is *Iota or Lambda* carageenan.

4. The hair-set composition according to Claim 1, wherein said ethylene oxide-addition type nonionic surface active agent has a melting point higher than 25° C and lower than 50° C and a viscosity smaller than 100,000 poise at 50° C.

5. The hair-set composition according to Claim 1, wherein said ethylene oxide-addition type nonionic surface active agent is a member selected from the group consisting of polyoxyethylene alkyl ethers, polyoxyethylene hydrogenated castor oils, polyethyleneglycol mono-fatty acid esters, and polyoxyethylene glyceryl mono-fatty acid esters.

6. The hair-set composition according to Claim 1, wherein said ethylene oxide-addition type nonionic surface active agent is a member selected from the group consisting of polyoxyethylene (EO = 10-25) lauryl ether, polyoxyethylene (EO = 5-15) cetyl ether, polyoxyethylene (EO = 5-10) stearyl ether, polyoxyethylene (EO = 5-25) hexyldecyl ether, polyoxyethylene (EO = 8-25) octyldodecyl ether, polyoxyethylene (EO = 10-30) isostearyl ether, polyoxyethylene (EO = 10-25) decyltetradecyl ether, polyoxyethylene (EO = 10-20) decylpentadecyl ether, polyoxyethylene (EO = 25-100) hydrogenated castor oil, polyethyleneglycol (EO = 5-25) monostearate, and polyoxyethylene (EO = 5-40) glyceryl monostearate.

7. The hair-set composition according to Claim 1, wherein said ethylene oxide-addition type nonionic surface active agent is a member selected from the group consisting of polyoxyethylene (EO = 5-25) hexyldecyl ether, polyoxyethylene (EO = 8-25) octyldodecyl ether, polyoxyethylene (EO = 10-30) isostearyl ether, and polyoxyethylene (EO = 25-100) hydrogenated castor oil.

8. The hair-set composition according to Claim 1, containing said component (a) in an amount of 0.2-5.0% by weight.

9. The hair-set composition according to Claim 1, containing said component (b) in an amount of 0.1-10.0% by weight.

10. The hair-set composition according to Claim 1, wherein the proportion of said components (a) and (b) is 1:30-5:1.